# EUROPEAN PATENT APPLICATION

(11) **EP 4 831 229 A1**
(43) Date of publication of application: **28.10.2026**
(21) Application number: 25804563.2
(22) Date of filing: 07.08.2025
(51) Int. Cl.: C08J 11/08, C08J 11/10, C07C 37/70, C07C 39/15, C08G 64/30, C08L 69/00

(54) **PROCESS FOR PURIFICATION OF WASTE POLYCARBONATE-BASED RESIN AND PREPARATION METHOD OF MONOMER FOR SYNTHESIZING RECYCLED PLASTIC, MONOMER FOR SYNTHESIZING RECYCLED PLASTIC, RECYCLED PLASTIC AND MOLDED PRODUCT, WHICH USE SAME**

(30) Priority: 20.02.2025 KR 20250022484
(71) Applicant: LG Chem, Ltd., Seoul 07336 (KR)
(72) Inventor: PARK, Tae Seung, Daejeon 34122 (KR); PARK, Eunju, Daejeon 34122 (KR); LEE, Jinhyeok, Daejeon 34122 (KR); HONG, Mooho, Daejeon 34122 (KR)
(74) Representative: Goddar, Heinz J.
(86) International application number: PCT/KR2025/011885
(87) International publication number: WO 2026/177284

(57) **Abstract**

The present invention relates to a process for purification of waste polycarbonate-based resin, comprising: a first step of injecting and filtering a precoat solution comprising polycarbonate-based resin, filter aid, and organic solvent into a filter; a second step, after the first step, of injecting and filtering a waste polycarbonate-based resin solution comprising waste polycarbonate-based resin, filter aid, and organic solvent into the filter; and a third step of obtaining a purified polycarbonate-based resin solution that has passed through the filter of the first and second steps; and to a preparation method of monomer for synthesising recycled plastic using the same, a monomer for synthesising recycled plastic, recycled plastic, and a molded article.

## Description

### [TECHNICAL FIELD]

### CROSS-REFERENCE TO RELATED APPLICATION(S)

This application claims the benefit of Korean Patent Application No. 10-2025-0022484 filed on February 20, 2025 with the Korean Intellectual Property Office, the disclosure of which is incorporated herein by reference in its entirety.

The present invention relates to a process for purification of waste polycarbonate-based resin, which removes impurities contained in waste polycarbonate-based resin to produce purified polycarbonate-based resin having high purity and improved color properties, and a method for manufacturing a monomer for recycled plastic synthesis using the same, a monomer for recycled plastic synthesis, recycled plastic, and a molded article.

### [BACKGROUND]

Polycarbonate is a thermoplastic polymer and is a plastic having excellent characteristics such as excellent transparency, superior ductility, and relatively low production costs.

Although polycarbonate is widely used for various purposes, environmental and health concerns during waste treatment have been continuously raised.

Currently, a physical recycling method is carried out, but in this case, a problem accompanying the deterioration of the quality occurs, and thus, research on the chemical recycling of polycarbonate is underway.

Chemical decomposition of polycarbonate refers to obtaining an aromatic diol compound as a monomer (e.g., bisphenol A (BPA)) through decomposition of polycarbonate, and then utilizing it again in polymerization to obtain a high-purity polycarbonate.

Meanwhile, waste polycarbonate contains various impurities, and in particular, impurities such as glass fiber and carbon black, which are insoluble in the solvent, are included. Therefore, before proceeding with chemical decomposition, a technology is used as a pretreatment process in which these impurities are removed by dissolving the waste polycarbonate in a solvent and then removing them through a filter.

However, when the pretreatment process of waste polycarbonate is carried out on a large scale, as the amount of impurities increases, a large amount of impurities covers the surface of the filter, causing the flow rate to drop sharply and shortening the replacement cycle of the filter, which presents a limitation.

Accordingly, there is a demand for the development of a technology that can further increase the filtration efficiency during the filter purification of waste polycarbonate.

### [DETAILED DESCRIPTION OF THE INVENTION]

### [Technical Problem]

The present invention is to provide a process for purification of waste polycarbonate-based resin, which can remove impurities contained in waste polycarbonate-based resin to manufacture a purified polycarbonate-based resin having high purity and improved color characteristics.

In addition, the present invention is to provide a preparation method of monomer for synthesising recycled plastic using the above process for purification of waste polycarbonate-based resin, a monomer for synthesising recycled plastic, recycled plastic, and a molded article.

### [Technical Solution]

In order to solve the above problem, in this specification, there is provided a process for purification of waste polycarbonate-based resin, comprising: a first step of injecting and filtering a precoat solution comprising polycarbonate resin, filter aid, and organic solvent into a filter; a second step, after the first step, of injecting and filtering a waste polycarbonate resin solution comprising waste polycarbonate resin, filter aid, and organic solvent into the filter; and a third step of obtaining a purified polycarbonate resin solution that has passed through the filter of the first and second steps.

This specification also provides a preparation method of monomer for synthesising recycled plastic, comprising a step of recovering an aromatic diol compound obtained by a depolymerization reaction of the purified polycarbonate-based resin solution obtained in the above-described process for purification of waste polycarbonate-based resin.

This specification also provides a recycled plastic comprising a reaction product of the above recycled plastic synthesis monomer and a comonomer.

This specification also provides a molded article comprising the above recycled plastic.

Hereinafter, the process for purification of waste polycarbonate-based resin according to specific embodiments of the invention, and the preparation method of monomer for synthesising recycled plastic using the same, the monomer for synthesising recycled plastic, the recycled plastic, and the molded article will be described in more detail.

Unless explicitly stated herein, the technical terms used herein are for the purpose of describing specific embodiments only and is not intended to limit the scope of the invention.

As used herein, the singular forms also include the plural forms unless the context clearly dictates otherwise.

The singular forms "a," "an" and "the" used herein are intended to include plural forms, unless the context clearly indicates otherwise.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present disclosure.

### 1. Process for purification of waste polycarbonate-based resin

According to one embodiment of the invention, a process for purification of waste polycarbonate-based resin may be provided, comprising: a first step of injecting and filtering a precoat solution comprising polycarbonate resin, filter aid, and organic solvent into a filter; a second step, after the first step, of injecting and filtering a waste polycarbonate resin solution comprising waste polycarbonate resin, filter aid, and organic solvent into the filter; and a third step of obtaining a purified polycarbonate resin solution that has passed through the filter of the first and second steps.

The inventors, as in the above embodiment of the process for purification of waste polycarbonate-based resin, have completed the invention by confirming through experiments that, when filtering waste polycarbonate-based resin through a filter, first filtering a precoat solution comprising polycarbonate-based resin, filter aid, and organic solvent to form a precoat layer on the surface of the filter membrane, and then filtering a waste polycarbonate-based resin solution comprising waste polycarbonate-based resin, filter aid, and organic solvent, can suppress the phenomenon of membrane pores being clogged by impurities and can improve filter performance.

In particular, since both the precoat solution and the waste polycarbonate-based resin solution comprise polycarbonate-based resin, by minimizing the concentration change of polycarbonate-based resin between the waste polycarbonate-based resin solution and the purified polycarbonate-based resin solution obtained after filter filtration, it is possible to proceed with the chemical decomposition process of polycarbonate-based resin using the purified polycarbonate-based resin solution without a separate concentration adjustment process, thereby improving process efficiency.

Specifically, the process for purification of waste polycarbonate-based resin according to the present embodiment may include a first step of introducing and filtering a precoat solution comprising polycarbonate-based resin, filter aid, and organic solvent into a filter. As in the above first step, a precoat solution comprising polycarbonate-based resin, filter aid, and organic solvent may be filtered first to form a precoat layer on the surface of the filter membrane. Accordingly, the precoat layer acts as a protective layer to suppress the phenomenon in which the membrane pores are blocked by impurities and to improve the filter performance.

The thickness of the precoat layer may be 25 mm or less, or may be 1 mm to 25 mm. If the thickness of the precoat layer excessively increases to exceed 25 mm, problems such as a decrease in filtration speed or damage and detachment of the precoat layer may occur.

The precoat solution may comprise polycarbonate-based resin, filter aid, and organic solvent.

The polycarbonate-based resin has a meaning that includes both a homopolymer or a copolymer containing polycarbonate repeating units, and refers collectively to the reaction product obtained through a polymerization reaction or copolymerization reaction of monomers including an aromatic diol compound and a carbonate precursor. In the case where only one kind of aromatic diol compound and one kind of carbonate precursor are used as the monomer to obtain only one kind of carbonate repeating unit, a homopolymer may be synthesized. In addition, when two or more kinds of carbonate precursors and one kind of aromatic diol compound are used as the monomer, or two or more kinds of aromatic diol compounds and one kind of carbonate precursor are used, or one or more kinds of other diols, in addition to one kind of aromatic diol compound and one kind of carbonate precursor, are used such that two or more kinds of carbonates are contained, a copolymer may be synthesized. The above-mentioned homopolymer or copolymer can include all of low molecular weight compounds, oligomers, and polymers according to molecular weight range.

The polycarbonate-based resin may be applied regardless of various forms and types, such as a new polycarbonate-based resin produced through synthesis, a recycled polycarbonate-based resin produced through a recycling process, or a polycarbonate-based resin waste. However, for a specific example, the polycarbonate-based resin contained in the precoat solution may be a new polycarbonate-based resin (neat PC), or a purified polycarbonate-based resin obtained in the purification method of the waste polycarbonate-based resin of the present embodiment.

The mass ratio of the polycarbonate-based resin in the pre-coat solution may be 10% to 20%. The method for measuring the mass ratio of the polycarbonate-based resin in the pre-coat solution may be obtained by dividing the mass of the polycarbonate-based resin by the total mass of the pre-coat solution and multiplying the resulting value by 100.

Since both the pre-coat solution and the waste polycarbonate-based resin solution, which will be described later, contain the polycarbonate-based resin, by minimizing the change in concentration of the polycarbonate-based resin between the waste polycarbonate-based resin solution and the purified polycarbonate-based resin solution obtained after filter filtration, it is possible to carry out the chemical decomposition process of the polycarbonate-based resin using the purified polycarbonate-based resin solution without a separate concentration adjustment process, thereby improving the efficiency of the process.

The filter aid may comprise at least one compound selected from the group consisting of diatomaceous earth, manganese sand, manganese zeolite, activated carbon, anthracite, ceramic sand, perlite, and cellulose. That is, the filter aid may comprise diatomaceous earth, manganese sand, manganese zeolite, activated carbon, anthracite, ceramic sand, perlite, cellulose, or a mixture of two or more thereof.

The above filter aid may have an average particle diameter of 10 µm to 15 µm. If the average particle diameter of the filter aid is excessively reduced, there is a problem in that the filtration rate becomes very slow, and if the average particle diameter of the filter aid is excessively increased, solid colorants among impurities may pass through the filtration filter and be discharged.

In the above precoat solution, the content of the filter aid, relative to 100 parts by weight of the polycarbonate resin, may be 1 part by weight to 20 parts by weight, or 10 parts by weight to 20 parts by weight, or 10 parts by weight to 5 parts by weight, or 11 parts by weight to 13 parts by weight. If the content of the filter aid is excessively reduced, it is difficult to sufficiently form a precoat layer on the surface of the filter membrane, so the effect of improving the filter performance is hard to achieve. On the other hand, if the content of the filter aid is excessively increased, there is a problem that the filtration rate decreases.

The above organic solvent may further comprise at least one organic solvents selected from the group consisting of tetrahydrofuran, toluene, methylene chloride, chloroform, dimethyl carbonate, ethyl methyl carbonate, diethyl carbonate, and dipropyl carbonate.

That is, the above organic solvent may comprise tetrahydrofuran, toluene, methylene chloride, chloroform, dimethyl carbonate, ethyl methyl carbonate, diethyl carbonate, dipropyl carbonate, or a mixture of two or more thereof.

More preferably, methylene chloride may be used as the above organic solvent. When methylene chloride is used as the above organic solvent, the solubility with respect to the polycarbonate resin or waste polycarbonate resin is improved, so the filtration performance by solid-liquid separation may be enhanced.

The filter is equipment for separating into a solid component and filtrate by pressure filtration, and the specific type of filter, the method of introducing into the filter, and the method of filtration in the filter are not particularly limited, and may be applied without limitation through various conventionally known filter technologies. However, as an example, a candle filter may be used.

Meanwhile, the process for purification of waste polycarbonate-based resin according to the above embodiment may include, after the first step, a second step of introducing and filtering a waste polycarbonate resin solution including waste polycarbonate resin, a filter aid, and an organic solvent into the above filter. Through the above second step, a large amount of impurities contained in the waste polycarbonate resin may be effectively removed, and a refined polycarbonate resin having high purity and improved color characteristics may be produced.

The waste polycarbonate resin solution may include waste polycarbonate resin, a filter aid, and an organic solvent.

Waste polycarbonate resin refers to waste of polycarbonate resin, and polycarbonate resin means both homopolymers or copolymers containing polycarbonate repeating units, and is a general term for reaction products obtained by a polymerization reaction or copolymerization reaction of monomers including an aromatic diol compound and a carbonate precursor. In the case where only one kind of aromatic diol compound and only one kind of carbonate precursor are used as monomers to obtain a polycarbonate repeating unit, a homopolymer may be synthesized. In addition, when two or more kinds of carbonate precursors are used with one kind of aromatic diol compound, or two or more kinds of aromatic diol compounds are used with one kind of carbonate precursor, or one or more kinds of other diols are used in addition to one kind of aromatic diol compound and one kind of carbonate precursor, a copolymer may be synthesized in which two or more kinds of carbonate repeating units are contained. The homopolymer or copolymer above may include all of a low molecular weight compound, oligomer, or polymer according to the molecular weight range.

The mass ratio of the waste polycarbonate resin in the waste polycarbonate resin solution may be 10% to 20%. The method for measuring the mass ratio of the waste polycarbonate resin in the waste polycarbonate resin solution may be calculated by dividing the mass of the waste polycarbonate resin by the total mass of the waste polycarbonate resin solution and multiplying the result by 100.

If the mass ratio of the waste polycarbonate resin in the waste polycarbonate resin solution is excessively decreased, the amount of organic solvent used increases, which is not advantageous in terms of economics, and if the mass ratio of the waste polycarbonate resin in the waste polycarbonate resin solution is excessively increased, there is a problem in that the filtration rate decreases.

The filter aid may comprise at least one compound selected from the group consisting of diatomaceous earth, manganese sand, manganese zeolite, activated carbon, anthracite, ceramic sand, perlite, and cellulose. That is, the filter aid may comprise diatomaceous earth, manganese sand, manganese zeolite, activated carbon, anthracite, ceramic sand, perlite, cellulose, or a mixture of two or more thereof.

The average particle size of the filter aid may be 10 µm to 15 µm. If the average particle size of the filter aid is excessively decreased, there is a problem in that the filtration rate becomes very slow, and if the average particle size of the filter aid is excessively increased, there is a possibility that solid colorant among the impurities may pass through the filtration filter and be discharged.

In the above waste polycarbonate-based resin solution, the content of the filter aid may be 1 part by weight to 20 parts by weight, or 1 part by weight to 10 parts by weight, or 1 part by weight to 5 parts by weight, or 2 parts by weight to 3 parts by weight, based on 100 parts by weight of the polycarbonate-based resin. If the content of the filter aid is excessively reduced, it is difficult to sufficiently form a filtration layer on the filter membrane, making it difficult to achieve the effect of improving filter performance. On the other hand, if the content of the filter aid is excessively increased, there is a problem in that the filtration lifespan becomes shortened.

The organic solvent may further include one or more organic solvents selected from the group consisting of tetrahydrofuran, toluene, methylene chloride, chloroform, dimethyl carbonate, ethylmethyl carbonate, diethyl carbonate, and dipropyl carbonate.

That is, the organic solvent may include tetrahydrofuran, toluene, methylene chloride, chloroform, dimethyl carbonate, ethylmethyl carbonate, diethyl carbonate, dipropyl carbonate, or a mixture of two or more thereof.

More preferably, methylene chloride may be used as the organic solvent. When methylene chloride is used as the organic solvent, the dissolution characteristics for the polycarbonate-based resin or waste polycarbonate-based resin are improved, thereby enhancing the filter filtration performance by solid-liquid separation.

Meanwhile, the waste polycarbonate-based resin solution may further include an adsorbent. The adsorbent, while serving as a filter aid, can remove organic dyes that impart color, thereby implementing a purified polycarbonate-based resin having high purity and improved color characteristics.

As examples of the above-mentioned adsorbent, activated carbon, charcoal, or mixtures thereof may be used. The above-mentioned activated carbon is a black carbon material having micropores, manufactured by subjecting a raw material to a carbonization process at about 500°C and an activation process (activated carbon) at about 900°C. Although the example is not particularly limited, for example, depending on the type of raw material, various types of activated carbon such as plant-based, coal-based, petroleum-based, and waste material activated carbon may be applied without limitation.

To give a more specific example, as plant-based activated carbon, coconut shell activated carbon, wood activated carbon, and sawdust activated carbon may be mentioned. Also, as coal-based activated carbon, lignite activated carbon, bituminous coal activated carbon, and anthracite activated carbon may be mentioned. Furthermore, as petroleum-based activated carbon, petroleum coke activated carbon and oil carbon activated carbon may be mentioned. Additionally, as waste material activated carbon, synthetic resin activated carbon and pulp activated carbon may be mentioned.

The above-mentioned adsorbent may include one or more activated carbons selected from the group consisting of plant-based activated carbon, coal-based activated carbon, petroleum-based activated carbon, and waste material activated carbon. That is, the above-mentioned adsorbent may include plant-based activated carbon, coal-based activated carbon, petroleum-based activated carbon, waste material activated carbon, or mixtures of two or more thereof.

More specifically, the above-mentioned adsorbent may include one or more activated carbons selected from the group consisting of coconut shell activated carbon, lignite activated carbon, anthracite activated carbon, and bituminous coal activated carbon. That is, the above-mentioned adsorbent may include coconut shell activated carbon, lignite activated carbon, anthracite activated carbon, bituminous coal activated carbon, or mixtures of two or more thereof.

The adsorption purification conditions by the above-mentioned adsorbent are not particularly limited, and various conventionally known adsorption purification conditions may be used without limitation. However, as an example, in the above-mentioned waste polycarbonate resin solution, the content of the adsorbent may be 0.1 parts by weight to 10 parts by weight, or 0.1 parts by weight to 5 parts by weight, or 0.5 parts by weight to 2 parts by weight, based on 100 parts by weight of the waste polycarbonate resin.

If the content of the above-mentioned adsorbent is excessively reduced, it is difficult to achieve a purified polycarbonate resin having high purity and improved color characteristics, as organic dyes expressing color cannot be sufficiently removed. On the other hand, if the content of the above-mentioned adsorbent is excessively increased, there is a problem in that the filtration rate decreases or the replacement cycle of the precoat becomes shorter.

In the above-mentioned waste polycarbonate resin solution, the content of the adsorbent may be 20 parts by weight to 500 parts by weight, or 40 parts by weight to 200 parts by weight, or 20 parts by weight to 80 parts by weight, or 40 parts by weight to 80 parts by weight, based on 100 parts by weight of the filter aid.

The above-mentioned filter is equipment for separating solid components and filtrate by pressure filtration, and the specific type of filter, the method of introducing into the filter, and the method of filtration in the filter are not particularly limited and may be applied without limitation through various conventionally known filter technologies. However, as an example, a candle filter may be used.

The filter of the second step is the same as the filter of the first step, and more specifically, the filter of the second step corresponds to a filter in which a precoat layer is formed on the surface of the filter membrane after proceeding with the first step.

Meanwhile, in the above step 2, the filtration rate according to the following Mathematical Formula 1 may be 100 L/m²h or more. [Mathematical Formula 1] 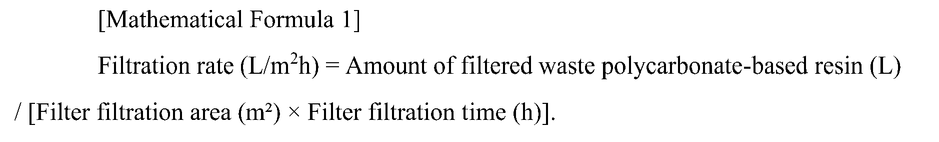 Filtration rate (L/m2h) = Amount of filtered waste polycarbonate-based resin (L) / [Filter filtration area (m2) × Filter filtration time (h)].

The process for purification of waste polycarbonate-based resin of this embodiment suppressed the phenomenon in which impurities cover the filter surface and the flow rate drops sharply, and the filtration rate according to Mathematical Formula 1 may be increased to 100 L/m²h or more. On the other hand, if the filtration rate according to Mathematical Formula 1 decreases to less than 100 L/m²h, it means that impurities derived from the waste polycarbonate-based resin cover the filter surface, causing a problem in which the flow rate drops sharply.

In addition, in the above step 2, the filtration lifespan according to the following Mathematical Formula 2 may be 50 kg/m² or more. [Mathematical Formula 2] 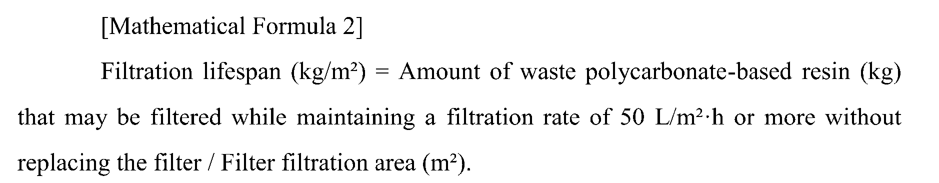 Filtration lifespan (kg/m2) = Amount of waste polycarbonate-based resin (kg) that may be filtered while maintaining a filtration rate of 50 L/m2·h or more without replacing the filter / Filter filtration area (m2).

As the filtration lifespan according to Mathematical Formula 2 increases to 50 kg/m² or more by the process for purification of waste polycarbonate-based resin of this embodiment, a high filtration rate may be maintained for a long time per unit filtration area, so the replacement cycle of the filter may be improved. On the other hand, if the filtration lifespan according to Mathematical Formula 2 decreases to less than 50 kg/m², it means that impurities derived from the waste polycarbonate-based resin cover the filter surface, causing a problem in which the flow rate drops sharply.

In addition, the process for purification of waste polycarbonate-based resin of this embodiment may have a polycarbonate concentration change according to the following Mathematical Formula 3 of 2% by weight or less, or 1% by weight or less, or 0.1% by weight or less, or 0% by weight or more, or 0% by weight to 2% by weight, or 0% by weight to 1% by weight, or 0% by weight to 0.1% by weight. [Mathematical Formula 3] 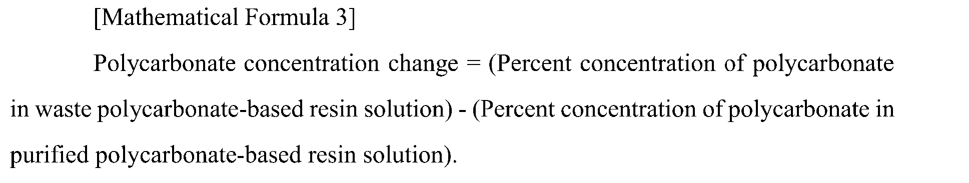 Polycarbonate concentration change = (Percent concentration of polycarbonate in waste polycarbonate-based resin solution) - (Percent concentration of polycarbonate in purified polycarbonate-based resin solution).

The process for purification of waste polycarbonate-based resin of the above embodiment allows the chemical decomposition process of the polycarbonate-based resin to be performed using the refined polycarbonate-based resin solution without a separate concentration adjustment process, as the polycarbonate concentration change according to Equation 3 becomes as low as 2% by weight or less, thereby increasing process efficiency. On the other hand, when the polycarbonate concentration change according to Equation 3 increases to more than 2% by weight, the process efficiency is poor because a separate process for additionally adjusting the concentration of the refined polycarbonate-based resin solution must be performed before proceeding with the chemical decomposition process of the polycarbonate-based resin.

Meanwhile, the second step may include a step 2-1 of introducing and circulating the waste polycarbonate-based resin solution containing the waste polycarbonate-based resin, filter aid, and organic solvent into the filter; and a step 2-2 of introducing and filtering the circulating liquid obtained in the step 2-1 into the filter. As described above, by first performing step 2-1 of introducing and circulating the waste polycarbonate-based resin solution containing the waste polycarbonate-based resin, filter aid, and organic solvent into the filter, a filtration layer may be formed on the filter membrane. Accordingly, the precote layer formed in the first step and the filtration layer together act as a protective layer, thereby suppressing the phenomenon of membrane pores being blocked by impurities and improving filter performance.

The step 2-1 of introducing and circulating the waste polycarbonate-based resin solution containing the waste polycarbonate-based resin, filter aid, and organic solvent into the filter may be performed for 30 minutes to 60 minutes. If the time of step 2-1 is excessively shortened, there is a problem in that it is difficult to sufficiently form a filtration layer on the filter membrane. On the other hand, if the time of step 2-1 is excessively increased, the process time becomes long and the process efficiency may decrease.

Meanwhile, in the step 2-2 of introducing and filtering the circulating liquid obtained in the step 2-1 into the filter, it may be performed one or more times, or two or more times, or three or more times as necessary so that the impurities contained in the circulating liquid are sufficiently removed. Step 2-2 may be performed for 30 minutes to 60 minutes. If the time of step 2-2 is excessively increased, the process time becomes long and the process efficiency may decrease.

In addition, the process for purification of waste polycarbonate-based resin of the above embodiment may include a third step of obtaining the refined polycarbonate-based resin solution that has passed through the filters of the first step and the second step.

The mixture of the precoat solution that has passed through the filter in the first step and the waste polycarbonate-based resin solution that has passed through the filter in the second step may be obtained as the refined polycarbonate-based resin solution.

Since both the precoat solution and the waste polycarbonate-based resin solution contain the polycarbonate-based resin at similar concentration ranges, it is possible to minimize the change in the concentration of the polycarbonate-based resin between the waste polycarbonate-based resin solution and the purified polycarbonate-based resin solution obtained after filter filtration, thereby enabling the chemical decomposition process of the polycarbonate-based resin to be carried out using the purified polycarbonate-based resin solution without a separate concentration adjustment process, which can increase the efficiency of the process.

The purity of the purified polycarbonate-based resin solution may be 99% or more. The method for measuring the purity of the purified polycarbonate-based resin solution is not particularly limited, and, for example, GC-MS, NMR, ICP-MS analysis, HPLC analysis, UPLC analysis, etc. may be used without limitation. Specific methods, conditions, equipment, etc. of the above GC-MS, NMR, ICP-MS, HPLC, and UPLC may be applied without limitation according to various contents known in the art.

As such, the purity of the purified polycarbonate-based resin solution is greatly increased to 99% or more, and by minimizing other impurities, the monomer recovered through chemical depolymerization using this can exhibit excellent physical properties.

In addition, the color coordinate L* of the purified polycarbonate-based resin solution may be 98 or more. In the present invention, the term "color coordinate" refers to the coordinate in the CIE Lab color space, which is a color value defined by the CIE (Commission Internationale de l'Eclairage), and any position in the CIE color space may be represented by the three coordinate values L*, a*, and b*.

Here, the L* value represents brightness, with L*=0 indicating black and L*=100 indicating white. Also, the a* value represents to what extent the color having the corresponding color coordinate is biased toward pure red or pure green, and the b* value represents to what extent the color having the corresponding color coordinate is biased toward pure yellow or pure blue.

If the color coordinate L* of the purified polycarbonate-based resin solution decreases to less than 98, the color characteristics of the purified polycarbonate-based resin solution become poor.

The method for measuring the color coordinate L* value of the purified polycarbonate-based resin solution is not particularly limited, and various color characteristic measuring methods in the field of plastics may be applied without limitation.

However, as an example of a method for measuring the color coordinate L* value, it may be measured in transmission mode using the HunterLab UltraScan PRO Spectrophotometer device.

### 2. Preparation method of monomer for synthesising recycled plastic

According to another embodiment of the invention, a preparation method of monomer for synthesising recycled plastic is provided, the method comprising a step of recovering an aromatic diol compound obtained by depolymerization reaction of the purified polycarbonate-based resin solution obtained by the process for purification of waste polycarbonate-based resin according to an embodiment.

The details of the process for purification of waste polycarbonate-based resin of the above embodiment, and the purified polycarbonate-based resin solution obtained thereby, all include the details described above in the embodiment.

The specific reaction conditions of the depolymerization reaction of the purified polycarbonate-based resin solution are not particularly limited, and the contents known in the prior art related to depolymerization of polycarbonate-based resins may be applied without limitation.

However, as an example, in the depolymerization reaction of the purified polycarbonate-based resin solution, the depolymerization reaction may be performed under acidic, neutral, or basic conditions, and in particular, the depolymerization reaction may be carried out under basic (alkaline) conditions. The type of the base is not particularly limited, and as an example, sodium hydroxide (NaOH) or potassium hydroxide (KOH) may be mentioned. The base acts as a base catalyst, and has the advantage of being more economical compared to organic catalysts that are mainly used under mild conditions. More specifically, in the depolymerization reaction of the purified polycarbonate-based resin solution, the depolymerization reaction may be carried out within a pH range of over 8 and less than 12.

In addition, the depolymerization reaction of the purified polycarbonate-based resin solution may be carried out in a solvent comprising ethanol. By decomposing the purified polycarbonate-based resin solution in a solvent containing ethanol, high-purity monomer bisphenol A may be stably obtained, and there is also the advantage that a high value-added byproduct, diethyl carbonate, may be additionally obtained.

The solvent in which the depolymerization reaction of the purified polycarbonate-based resin solution is carried out, in addition to ethanol, may further comprise at least one organic solvent selected from the group consisting of tetrahydrofuran, toluene, methylene chloride, chloroform, dimethyl carbonate, ethyl methyl carbonate, diethyl carbonate, and dipropyl carbonate.

More preferably, methylene chloride may be used as the organic solvent. When methylene chloride is used as the organic solvent to be mixed with ethanol, there is the advantage that the solubility for polycarbonate is improved, thereby enhancing reactivity.

Meanwhile, the temperature at which the depolymerization reaction of the purified polycarbonate-based resin solution is carried out is not particularly limited, but, for example, it may be carried out at 20°C to 100°C, or at 50°C to 70°C. In addition, the time for performing the depolymerization reaction of the purified polycarbonate-based resin solution may be 1 hour to 30 hours, or 4 hours to 6 hours.

Meanwhile, during the depolymerization reaction of the above-mentioned purified polycarbonate-based resin solution, an antioxidant may be added to the reaction solution. By adding the antioxidant, the aromatic diol compound recovered through recycling by chemical decomposition of the polycarbonate-based resin can satisfy a low color coordinate b* value, at a color level equivalent to commercially sold reagents or reagents used for PC polymerization.

Specific examples of the above-mentioned aromatic diol compound include bis(4-hydroxyphenyl)methane, bis(4-hydroxyphenyl)ether, bis(4-hydroxyphenyl)sulfone, bis(4-hydroxyphenyl)sulfoxide, bis(4-hydroxyphenyl)sulfide, bis(4-hydroxyphenyl)ketone, 1,1-bis(4-hydroxyphenyl)ethane, 2,2-bis(4-hydroxyphenyl)propane (bisphenol A), 2,2-bis(4-hydroxyphenyl)butane, 1,1-bis(4-hydroxyphenyl)cyclohexane (bisphenol Z), 2,2-bis(4-hydroxy-3,5-dibromophenyl)propane, 2,2-bis(4-hydroxy-3,5-dichlorophenyl)propane, 2,2-bis(4-hydroxy-3-bromophenyl)propane, 2,2-bis(4-hydroxy-3-chlorophenyl)propane, 2,2-bis(4-hydroxy-3-methylphenyl)propane, 2,2-bis(4-hydroxy-3,5-dimethylphenyl)propane, 1,1-bis(4-hydroxyphenyl)-1-phenylethane, or mixtures of two or more thereof, and the like. Preferably, the aromatic diol compound of the monomer for synthetic recycled plastic of this embodiment may be 2,2-bis(4-hydroxyphenyl)propane (bisphenol A).

### 3. Monomer for synthesising recycled plastic

According to another embodiment of the invention, a monomer for synthesising recycled plastic, which comprises an aromatic diol compound obtained from the preparation method of monomer for synthesising recycled plastic of the other embodiment, may be provided.

That is, the monomer for synthesizing recycled plastic of the other embodiment may be obtained by the method for preparing a monomer for synthesizing recycled plastic. The contents regarding the preparation method of monomer for synthesising recycled plastic include all the descriptions previously mentioned in the other embodiment.

The aromatic diol compound is characterized by being obtained from the preparation method of monomer for synthesising recycled plastic of the other embodiment. That is, the aromatic diol compound is characterized by being recovered from the purified polycarbonate-based resin used in the recovery of the monomer for synthesizing recycled plastic. Therefore, cases in which a new aromatic diol compound is externally added, separately from the recovery from the purified polycarbonate-based resin for manufacturing the monomer for synthesizing recycled plastic of the other embodiment, are not included in the category of aromatic diol compounds of the present invention.

Specifically, being recovered from the polycarbonate-based resin means that it is obtained through the depolymerization reaction of the polycarbonate-based resin. The depolymerization reaction may be performed under acidic, neutral, or basic conditions, and in particular, the depolymerization reaction may be carried out under basic (alkaline) conditions. Particularly, as will be described later, it is preferable that the depolymerization reaction is carried out in an ethanol solvent.

The monomer for synthesizing recycled plastic of the other embodiment may be used as a raw material for manufacturing various recycled plastics (for example, polycarbonate (PC)) as will be described later.

The monomer for synthesizing recycled plastic of the other embodiment may further include small amounts of other additives or solvents, and the specific types of additives or solvents are not particularly limited, and various substances widely used in the process for recovering aromatic diol compounds by depolymerization of polycarbonate-based resin may be applied without limitation.

### 4. Recycled Plastic

According to another embodiment of the invention, recycled plastic comprising a reaction product of the monomer for synthesising recycled plastic of the above-described other embodiment and comonomer may be provided.

All of the contents regarding the monomer for synthesising recycled plastic of the other embodiment described above are included in this embodiment.

Examples corresponding to the recycled plastic are not particularly limited, and various plastics synthesized using an aromatic diol compound such as bisphenol A and a carbonate precursor such as dimethyl carbonate, diethyl carbonate, or ethyl methyl carbonate as a monomer may be applied without limitation, and more specifically, polycarbonate resin may be given as an example.

More specifically, in the recycled plastic comprising the reaction product of the monomer and comonomer for recycled plastic synthesis, a carbonate precursor may be used as the comonomer. Specific examples of the carbonate precursor include phosgene, triphosgene, diphosgene, bromophosgene, dimethyl carbonate, diethyl carbonate, dibutyl carbonate, dicyclohexyl carbonate, diphenyl carbonate, ditolyl carbonate, bis(chlorophenyl) carbonate, m-cresyl carbonate, dinaphthyl carbonate, bis(diphenyl) carbonate, or bishaloformate.

The examples of the reaction process of the monomer and comonomer for recycled plastic synthesis used to synthesize the polycarbonate resin are not particularly limited, and various conventionally known polycarbonate manufacturing methods may be applied without limitation.

However, as an example of the polycarbonate manufacturing method, a polycarbonate manufacturing method including a step of polymerizing a composition including the monomer and comonomer for recycled plastic synthesis may be used. At this time, the polymerization may be performed by interfacial polymerization, and in the case of interfacial polymerization, the polymerization reaction may be performed at atmospheric pressure and low temperature, and molecular weight control is easy.

The polymerization temperature may be 0°C to 40°C, and the reaction time may be 10 minutes to 5 hours. In addition, during the reaction, the pH may be maintained at 9 or higher, or 11 or higher.

As the solvent usable for the polymerization, any solvent used in the art for the polymerization of polycarbonate may be used without particular limitation, and as an example, halogenated hydrocarbons such as methylene chloride and chlorobenzene may be used.

In addition, the polymerization may be performed in the presence of an acid acceptor, and as the acid acceptor, an alkali metal hydroxide such as sodium hydroxide or potassium hydroxide, or an amine compound such as pyridine may be used.

In addition, in order to control the molecular weight of the polycarbonate during the polymerization, the polymerization may be performed in the presence of a molecular weight regulator. As the molecular weight regulator, an alkylphenol having 1 to 20 carbon atoms may be used, and specific examples thereof include p-tert-butylphenol, p-cumylphenol, decylphenol, dodecylphenol, tetradecylphenol, hexadecylphenol, octadecylphenol, eicosylphenol, docosylphenol, or triacontylphenol. The molecular weight regulator may be added before the initiation of polymerization, during the initiation of polymerization, or after the initiation of polymerization. The molecular weight regulator may be used in an amount of 0.01 to 10 parts by weight, or 0.1 to 6 parts by weight, based on 100 parts by weight of the aromatic diol compound, and the desired molecular weight may be obtained within this range.

In addition, in order to promote the polymerization reaction, a reaction promoter such as a tertiary amine compound, a quaternary ammonium compound, or a quaternary phosphonium compound, including triethylamine, tetra-n-butylammonium bromide, or tetra-n-butylphosphonium bromide, may be further used.

### 5. Molded Article

According to another embodiment of the invention, a molded article comprising the recycled plastic of the above-described other embodiment may be provided. The content regarding the recycled plastic includes all matters described in detail in the above-mentioned other embodiment.

The molded article may be obtained by applying, without limitation, various known plastic molding methods to the recycled plastic, and examples of the molding methods include, but are not limited to, injection molding, foam injection molding, blow molding, or extrusion molding.

The examples of the molded article are not significantly limited, and it may be applied without limitation to various molded articles using plastics. Examples of the molded article include, but are not limited to, automobile parts, electric and electronic products, communication products, household goods, building materials, optical components, and exterior materials.

The molded article, in addition to the recycled plastic of the above-described other embodiment, may further comprise, as necessary, at least one additive selected from the group consisting of antioxidant, plasticizer, antistatic agent, nucleating agent, flame retardant, lubricant, impact modifier, fluorescent whitening agent, ultraviolet absorber, pigment, and dye.

As an example of a method for manufacturing the molded article, the method may comprise mixing the recycled plastic of the above-described other embodiment and additives well using a mixer, then manufacturing pellets by extrusion molding with an extruder, drying the pellets, and then injecting them with an injection molding machine.

### [Advantageous Effects]

According to the present invention, there may be provided a method for purifying waste polycarbonate, which removes impurities contained in waste polycarbonate to produce purified polycarbonate having high purity and improved color characteristics, a method for preparing a monomer for recycled plastic synthesis using the same, a monomer for recycled plastic synthesis, recycled plastic, and a molded article.

### [DETAILED DESCRIPTION OF THE EMBODIMENTS]

Hereinafter, the present disclosure will be explained in detail with reference to the following examples. However, these examples are for illustrative purposes only, and the scope of the present disclosure is not limited thereto.

### <EXAMPLE and COMPARATIVE EXAMPLE>

### Example 1

0.6 kg of waste polycarbonate (w-PC), 12 g of diatomaceous earth (average particle diameter 13 µm), and 6 g of charcoal were dissolved in 3.4 kg of methylene chloride (MC) to prepare a w-PC solution.

0.3 kg of transparent polycarbonate (neat PC) and 36 g of diatomaceous earth (average particle diameter 13 µm) were dissolved in 1.7 kg of methylene chloride (MC) to prepare a pre-coat solution.

Using a pump, the pre-coat solution was introduced into a candle filter and filtered inside the filter at room temperature for 30 minutes to form a pre-coat layer on the surface of the filter membrane.

Thereafter, the w-PC solution was introduced into the candle filter and filtered inside the filter for 30 minutes to perform pre-filtration.

Thereafter, the filtered solution was circulated again into the candle filter, and filtered inside the filter for 30 minutes to perform main-filtration.

Thereafter, the filtrate, which is the purified polycarbonate (r-PC) solution, was recovered.

In addition, the inside of the candle filter was drained to recover the remaining solution, and the filtercake was recovered.

### Example 2

Except that the w-PC solution in Example 1 was prepared by dissolving 0.6 kg of waste polycarbonate (w-PC) and 18 g of diatomaceous earth (average particle diameter 13 µm) in 3.4 kg of methylene chloride (MC), the purified polycarbonate (r-PC) solution was recovered in the same manner as in Example 1.

### Comparative Example 1

Except that a solution prepared by dissolving 36 g of diatomaceous earth (average particle diameter 13 µm) in 2 kg of methylene chloride (MC) was used as the pre-coat solution, the purified polycarbonate (r-PC) solution was recovered in the same manner as in Example 1.

### <EXPERIMENTAL Example>

With respect to the purified polycarbonate (r-PC) solutions obtained in the above Examples and Comparative Example, physical properties were measured by the following method, and the results are shown in Table 1.

### 1. Purity

1 g of the purified polycarbonate (r-PC) solution obtained in the above Example and Comparative Example was dissolved in 10 mL of chloroform, and then 20 mL of methanol was added to the solution to precipitate the polymer component. Thereafter, after complete precipitation using a centrifuge, 10 mL of the supernatant was transferred to a vial and completely dried under N2 purge. After adding 1 mL of a mixed solvent of chloroform:acetone = 1:2 to the vial and completely dissolving it, it was filtered, and a gas chromatography-mass spectrometry (GC-MS) spectrum was obtained under the following conditions using GC/MS equipment, and the purity of the purified polycarbonate (r-PC) was analyzed.

### <GAS Chromatography (GC) Conditions>

① Column: HP-5MS(L:30m, ID:0.25mm, film:0.25µm)
② Injection volume: 0.2*µℓ*
③ Inlet, Temp.: 300 °C, spilt ratio: 20:1
④ Column flow: (He) 1ml/min
⑤ Oven temp.: 50°C/5min-10°C/min-320°C/15min (Total 47min)
⑥ Detector Temp.:300°C
⑦ GC Model: Agilent 7890

### 2. Color Coordinate (L*)

For the solution in which the concentration of purified polycarbonate (r-PC) solution obtained from the above Example and the purified polycarbonate (r-PC) solution obtained from the above Comparative Example was adjusted to be 15% by weight, the color coordinate (L*) was measured in transmission mode using the HunterLab UltraScan PRO Spectrophotometer equipment.

### 3. Filtration Performance

### (1) Filtration Rate (L/m²h)

In the main-filtration process, the filtration amount (L) of w-PC, filter filtration area (m²), and filter filtration time (h) were measured, and the filtration rate was calculated according to the following Mathematical Formula 1. [Mathematical Formula 1] 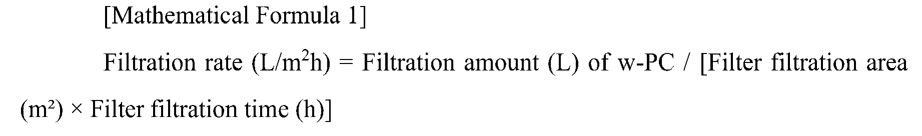 Filtration rate (L/m2h) = Filtration amount (L) of w-PC / [Filter filtration area (m2) × Filter filtration time (h)]

### (2) Filtration Lifetime (kg/m²)

In the above main-filtration process, the filtration lifetime was determined according to the following Mathematical Formula 2 by measuring the amount of w-PC (kg) that may be filtered while maintaining a filtration rate of 50 L/m²h or higher without filter replacement, and the filter filtration area (m²). [Mathematical Formula 2] 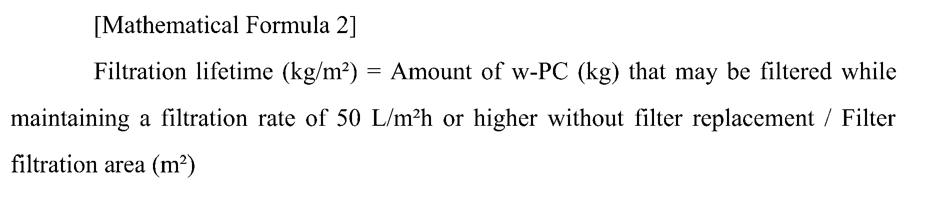 Filtration lifetime (kg/m2) = Amount of w-PC (kg) that may be filtered while maintaining a filtration rate of 50 L/m2h or higher without filter replacement / Filter filtration area (m2)

### 4. Change in Polycarbonate Concentration

The difference between the percent concentration (weight%) of polycarbonate in the waste polycarbonate (w-PC) solution and the percent concentration (weight%) of polycarbonate in the purified polycarbonate (r-PC) solution was calculated using the following Mathematical Formula 3. [Mathematical Formula 3] 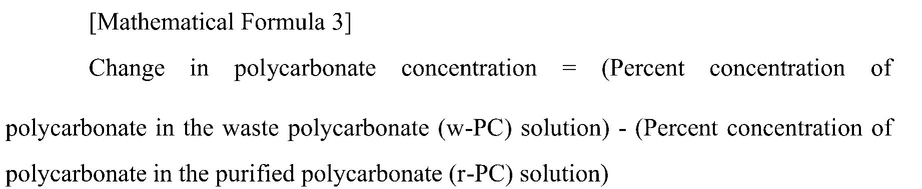 Change in polycarbonate concentration = (Percent concentration of polycarbonate in the waste polycarbonate (w-PC) solution) - (Percent concentration of polycarbonate in the purified polycarbonate (r-PC) solution)

**[Table 1]**

| Experimental Example Measurement Results | | | | | |
|---|---|---|---|---|---|
| Category | Purity (%) | Color Coordinate (L*) | Filtration Rate (L/m²h) | Filtration Lifetime (kg/m²) | Change in Polycarbonate Concentration (wt%) |
| Example 1 | 99.9 | 99.79 | 120 | 50 or more | 0 |
| Example 2 | 99.9 | 98.89 | 120 | 50 or more | 0 |
| Comparative Example 1 | 99.9 | 99.79 | 150 | 50 or more | 4.5 |

According to Table 1 above, the purified polycarbonate obtained in the Example exhibited high purity equal to or higher than that of the Comparative Example, excellent color properties, and filtration properties, and also had the advantage of exhibiting little change in polycarbonate concentration, so that it could be applied to the polycarbonate depolymerization process without a separate concentration control process.

## Claims

1. A process for purification of waste polycarbonate-based resin comprising:
a first step of injecting and filtering a precoat solution comprising polycarbonate-based resin, filter aid, and organic solvent into a filter;
a second step, after the first step, of injecting and filtering a waste polycarbonate-based resin solution comprising waste polycarbonate-based resin, filter aid, and organic solvent into the filter; and
a third step of obtaining a purified polycarbonate-based resin solution that has passed through the filter of the first and second steps.

2. The process for purification of waste polycarbonate-based resin according to claim 1,
wherein, in the second step,
the filtration rate according to the following mathematical formula 1 is 100 L/m²h or more: [Mathematical Formula 1] 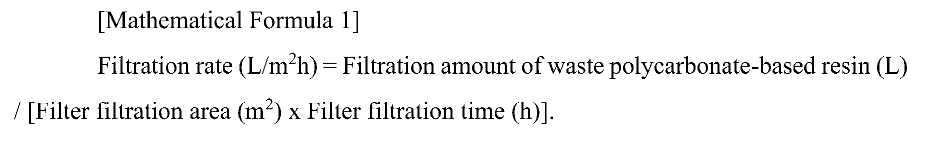 Filtration rate (L/m2h) = Filtration amount of waste polycarbonate-based resin (L) / [Filter filtration area (m2) x Filter filtration time (h)].

3. The process for purification of waste polycarbonate-based resin according to claim 1,
wherein, in the second step,
the filtration lifetime according to the following mathematical formula 2 is 50 kg/m² or more: [Mathematical Formula 2] 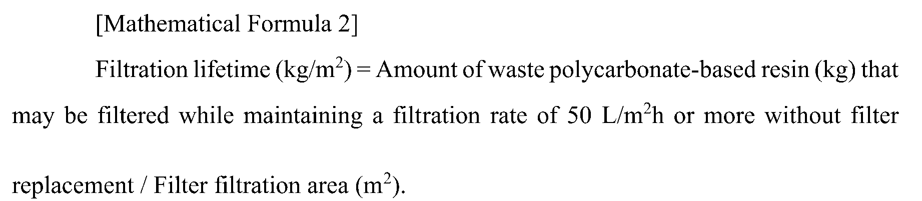 Filtration lifetime (kg/m2) = Amount of waste polycarbonate-based resin (kg) that may be filtered while maintaining a filtration rate of 50 L/m2h or more without filter replacement / Filter filtration area (m2).

4. The process for purification of waste polycarbonate-based resin according to claim 1,
wherein the change in polycarbonate concentration according to the following mathematical formula 3 is 2 wt% or less: [Mathematical Formula 3] 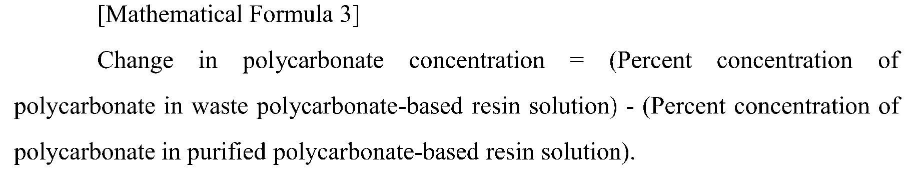 Change in polycarbonate concentration = (Percent concentration of polycarbonate in waste polycarbonate-based resin solution) - (Percent concentration of polycarbonate in purified polycarbonate-based resin solution).

5. The process for purification of waste polycarbonate-based resin according to claim 1,
wherein the purity of the purified polycarbonate-based resin solution is 99% or more.

6. The process for purification of waste polycarbonate-based resin according to claim 1,
wherein the color coordinate L* of the purified polycarbonate-based resin solution is 98 or more.

7. The process for purification of waste polycarbonate-based resin according to claim 1,
wherein the second step comprises:
a second-1 step of injecting and circulating the waste polycarbonate-based resin solution comprising waste polycarbonate-based resin, the filter aid, and the organic solvent into the filter; and
a second-2 step of injecting and filtering the circulating liquid obtained from the second-1 step into the filter.

8. The process for purification of waste polycarbonate-based resin according to claim 1, wherein the organic solvent is methylene chloride.

9. The process for purification of waste polycarbonate-based resin according to claim 1,
wherein the mass ratio of the waste polycarbonate-based resin in the waste polycarbonate-based resin solution is 10% to 20%.

10. The process for purification of waste polycarbonate-based resin according to claim 1,
wherein the mass ratio of the polycarbonate-based resin in the precoat solution is 10% to 20%.

11. The process for purification of waste polycarbonate-based resin according to claim 1,
wherein, in the waste polycarbonate-based resin solution, the content of the filter aid is 1 part by weight to 20 parts by weight based on 100 parts by weight of the polycarbonate-based resin.

12. The process for purification of waste polycarbonate-based resin according to claim 1, wherein the filter aid comprises at least one compound selected from the group consisting of diatomaceous earth, manganese sand, manganese zeolite, activated carbon, anthracite, ceramic sand, perlite, and cellulose.

13. The process for purification of waste polycarbonate-based resin according to claim 1, wherein the filter aid has an average particle diameter in range of 10 µm to 15 µm.

14. The process for purification of waste polycarbonate-based resin according to claim 1,
wherein, in the precoat solution, the content of the filter aid is in range of 1 part by weight to 20 parts by weight based on 100 parts by weight of the polycarbonate-based resin.

15. The process for purification of waste polycarbonate-based resin according to claim 1,
wherein the waste polycarbonate-based resin solution further comprises an adsorbent.

16. The process for purification of waste polycarbonate-based resin according to claim 1, wherein the adsorbent comprises charcoal or activated carbon.

17. A preparation method of monomer for synthesising recycled plastic, comprising a step of recovering an aromatic diol compound obtained by a depolymerization reaction of the purified polycarbonate-based resin solution obtained from the process for purification of waste polycarbonate-based resin according to claim 1.

18. A monomer for synthesising recycled plastic comprising the aromatic diol compound obtained in the method for preparing a monomer for synthesising recycled plastic of claim 17.

19. A recycled plastic comprising a reaction product of the monomer for synthesising recycled plastic of claim 18 and a comonomer.

20. A molded article comprising the recycled plastic of claim 19.
